# EUROPÄISCHE PATENTSCHRIFT

(11) **EP 1 711 293 B1**
(45) Veröffentlichungstag und Bekanntmachung des Hinweises auf die Patenterteilung: **01.04.2009**
(21) Anmeldenummer: 04765697.0
(22) Anmeldetag: 30.09.2004
(51) Int. Cl.: B22F 3/24

(54) **SAUERSTOFF-ANGEREICHERTE REFRAKTÄRMETALL-LEGIERUNG FÜR DIE MEDIZINTECHNIK**
METALLIC MATERIAL, METHOD FOR THE PRODUCTION THEREOF, AND USE OF THE SAME
MATERIAU METALLIQUE, SON PROCEDE DE PRODUCTION ET SON UTILISATION

(30) Priorität: 17.10.2003 DE 10349102; 01.07.2004 DE 102004032128
(43) Veröffentlichungstag der Anmeldung: 18.10.2006
(73) Patentinhaber: W.C. Heraeus GmbH, 63450 Hanau (DE)
(72) Erfinder: TRÖTZSCHEL, Jens, 63486 Bruchköbel (DE); SPANIOL, Bernd, 63546 Hammersbach (DE)
(74) Vertreter: Kühn, Hans-Christian
(86) Internationale Anmeldenummer: PCT/EP2004/010904
(87) Internationale Veröffentlichungsnummer: WO 2005/037468

(56) Entgegenhaltungen:
- WO-A-03/008657
- WO-A2-02/098275
- US-A- 2 987 352
- US-A1- 2004 149 356

## Beschreibung

Die Erfindung betrifft die Verwendung eines metallischen Werkstoffs aus Niob oder einer Legierung auf Basis von Niob.

Insbesondere im Bereich der Medizintechnik werden Drähte, Rohre oder Bänder aus Edelmetallen (z.B. Platin oder Platinlegierungen) oder aus Refraktärmetaiten (Molybdän, Niob, Rhenium, Tantal, Titan, Wolfram, Zirkonium) und deren Legierungen (beispielsweise NbZr1 oder TaNbxYz) sowie Edelstähle oder Nitinol für die unterschiedlichsten Anwendungen eingesetzt. Die meisten metallischen Werkstoffe, die für die unterschiedlichsten medizinischen Anwendungen zum Einsatz kommen, stellen einen Kompromiss dar zwischen guter Kompatibilität. Handhabbarkeit, guter mechanischer Eigenschaften, Verarbeitbarkeit und den damit in Zusammenhang stehenden Kosten. Die Anforderungen an Materialien, beispielsweise für Stents, sind vielschichtig. Es wird nach Materialien mit einer möglichst hohen Festigkeit gesucht. Durch eine hohe Festigkeit können die benötigen Stegquerschnitte entsprechend auf ein Minimum verringert werden. Das Kriterium ist hierbei eine möglichst hohe Stützwirkung mit möglichst geringer in den menschlichen Körper einzubringender Materialmenge. Die Reduzierung der Metallmenge hat auch zur Folge, dass die Kernspinkompatibilität von Stents verbessert wird, da weniger Material auch weniger Artefakte bei der Untersuchung im Kemspintomografen erzeugt. Gleichzeitig mit der Reduzierung der Materialmenge verschlechtert sich aber die Sichtbarkeit der Teile (z. B. Stents) im Röntgenbild. Dieser Nachteil kann umgangen werden, indem eine Beschichtung aus einem gut röntgensichtbaren Material (in der Regel ein Edelmetall mit hoher Ordnungszahl) auf die Struktur aufgebracht wird oder es mit sogenannten Markem versehen wird. Bei beiden Varianten besteht ein gewisses Risiko, das sich bei der Applikation Korrosionspotentiale zwischen den unterschiedlichen Materialien ausbilden, die zur Schwächung oder Auflösung der betroffenen Teile führen können.

Einige bekannte Materialien für Stents (Edelstahl, CoCr-Legierungen, MP35N oder Nitinol) sind in der Regel nicht hilfreich bzw. enthalten weitere chemische Bestandteile, die als nicht biokompatibel angesehen werden.

Refraktärmetalle weisen einen relativ guten Röntgenkontrast auf. Sie sind zudem in der Regel vollständig ineinander löslich, wodurch die Möglichkeit besteht, den Röntgenkontrast zu variieren und gezielt einzustellen.

Metallische Werkstoffe der eingangs charakterisierten Art sind beispielsweise aus US 6,358,625 B1 bekannt. Hier sind Anodendrähte aus Niob oder Tantal offenbart, die zur Verbesserung der Haftung derart mit Sauerstoff behandelt werden, dass sich eine Anreicherung an der Oberfläche in der Größenordnung von 35 Atomprozent in einer Schicht von etwa 50 nm ergibt. Diese Anreicherung führt zu einer Oberflächenschicht mit Nioboxid bzw. Tantaloxid, welches als Sinterhilfe bei der Herstellung von Kondensatoren dient

Aus Espe, Werkstoffkunde der Hochvakuumtechnik, Bd. 1, VEB Deutscher Verlag der deutschen Wissenschaften Berlin, 1959, Seiten 146 bis 149, ist eine oberflächliche Oxidation von Niob bekannt, wobei eine Versprödung des Materials mit steigender Sauerstoffaufnahme einsetzt, wobei also die Dehnung des Materials abnimmt. Insbesondere ergibt sich die Abnahme der Bruchdehnung aus Tabelle T3.7-2b. Daher stellt diese Lehre auch darauf ab, den Sauerstoff zu entfernen;

Aus US 5,242,481 sind, in Übereinstimmung mit der Offenbarung in Espe, pulvermetallurgisch hergestellte Erzeugnisse aus Tantal oder Niob bekannt, die einen Sauerstoffgehalt von weniger als 300 ppm aufweisen. Ebenfalls in Übereinstimmung damit ist aus DE 37 00 659 A1 bekannt, dass Tantal versprödet, wenn es sauerstoffhaltige Atmosphäre bei höheren Temperaturen ausgesetzt wird. Dadurch verliert es seine Dehnbarkeit. Aus WO 03/008657 A1 ist ein medizinisches Implantat mit einer oxidierten Zirkonbeschichtung bekannt.

Aufgabe der vorliegenden Erfindung ist es, die Eigenschaften der bekannten Werkstoffe aus Niob oder einer Legierung auf Basis von Niob, insbesondere ihre mechanischen Eigenschaften, wie Dehnbarkeit, zu verbessern, sowie eine geeignete Verwendung dieser Werkstoffe anzugeben.

Die Aufgabe wird erfindungsgemäß durch die Merkmale desf unabhängigen Anspruchs gelöst. Vorteilhafte Ausgestaltungen der Erfindung sind in den abhängigen Ansprüchen angegeben. Dadurch, dass der metallische Werkstoff aus Niob oder einer Legierung auf Basis von Niob einen Sauerstoffgehalt von 1.000 bis 30.000 µg/g aufweist und dass der Sauerstoff interstitiell in der Gitterstruktur des Metalls eingelagert ist, ergeben sich überraschenderweise gute mechanische Kennwerte (hohe Zugfestigkeit und Dehnung) bei Raumtemperatur oder in der Nähe der Raumtemperatur trotz (oder gerade wegen) des hohen Sauerstoffgehaltes, der nach der bisherigen Lehrmeinung und allgemeinen Erfahrung eher zu einer Versprödung des Materials und somit zu einer Reduzierung der Duktilität führen würde. Diese bisherige Annahme ist u. a. der Grund dafür, dass die für die Anwendungen von Refraktärmetallen in der Medizintechnik anzuwendenden Normen ASTMB362 (für Niob und NbZr1) und ASTMF560 (für Tantal) den maximal zulässigen Sauerstoffgehalt begrenzen auf Werte zwischen 100 und 400 ppm. Die entsprechenden Herstellverfahren für derartige Metalle sind demzufolge so ausgelegt, dass eine Sauerstoffaufnahme des Materials während der Bearbeitung grundsätzlich ausgeschlossen ist. Die nunmehr gefundene Lehre setzt sauerstoffgezielt zur Eigenschaftsverbesserung ein und eröffnet damit die Möglichkeit, das Anwendungsspektrum von Refraktärmetallen und deren Legierung in der Medizintechnik und in anderen Bereichen erheblich zu erweitern. Zur Erklärung des erzielten Effekts wird angenommen, dass die interstitiellen Einlagerungen die Beweglichkeit von Versetzungen drastisch herabsetzen. Damit kommt es im Weiteren wahrscheinlich schon während der plastischen Deformation zur Bildung einer erhöhten Versetzungsdichte innerhalb der Körner des vor der Verformung vorhandenen Gefüges. Die Anzahl dieser Versetzungen, der Umformgrad und der Sauerstoffgehalt bzw. der Gehalt an sonstigen interstitiellen Verunreinigungen oder Dotierungen beeinflussen die späteren mechanischen Eigenschaften. Bei späterer Rekristallisation werden dabei zusätzliche Keime für das Komwachstum gebildet und so ein für die mechanischen Eigenschaften vorteilhaftes feinkörniges Gefüge generiert. Dazu ist es vorteilhaft, dass der Sauerstoff homogen verteilt eingelagert ist. Zweckmäßig ist ein Sauerstoffgehalt von 1.000 bis 15.000, vorzugsweise bis 8.000 mg/g. kann mit einem weiteren Refraktärmetall, insbesondere auch aus dieser Gruppe Titan, Zirkonium, Molybdän, Tantal, Wolfram, Rhenium, legiert sein. Der metallische Werkstoff kann mit einem oder mehreren chemischen Elementen aus der Gruppe H, C, N, P, S, V, Y, La, Hf, Ce, Th dotiert sein. Zweckmäßig ist es, dass der Werkstoff eine Zugfestigkeit von mindestens 700 mPa bei einer Dehnung von etwa 20 bis 30 % aufweist.

Der Werkstoff kann durch ein Verfahren hergestellt werden, bei dem er bei etwa 600 bis 800 °C und einem Sauerstoffpartialdruck von < 5 mbar mit Sauerstoff angereichert wird. Insbesondere kann der Vorgang in einem sauerstoffhaltigen Gas erfolgen. Der Werkstoff kann auch dadurch hergestellt werden, dass auf oder an seiner Oberfläche eine, vorzugsweise amorphe, Oxidschicht gebildet wird und dass danach ein Diffusionsglühen erfolgt, bei dem der Sauerstoff in die Tiefe des Werkstoffes eindringt. Amorphe Oxidschichten diffundieren leichter in das Material ein, die Diffusion sollte zumindest gleichschnell wie die Oxidbildung an der Oberfläche erfolgen. Dabei ist es vorteilhaft, dass die Oxidschicht nass-chemisch, insbesondere durch anodische Oxidation oder thermisch, insbesondere durch Oberflächenoxidation unter Sauerstoffatmosphäre gebildet wird. Alternativ kann der Werkstoff auch dadurch hergestellt werden, dass das Refraktärmetall oder die Legierung durch einen Sintervorgang gebildet wird, wobei Sauerstoff eingebracht wird. Dies ist z. 8. möglich bei Sintern von oxidischem Pulver. Vorzugsweise wird der metallische Werkstoff nach der Sauerstoffanreicherung einer Umformung unterworfen, wobei die Umformung vorzugsweise bis an den kritischen Umformungsgrad heran erfolgt. Der kritsche Umformungsgrad ist der für jeden Werkstoff spezifische Umformgrad, der mindestens notwendig ist, um bei einer Wärmebehandlung Sekundärkristallisation (Kornneubildung) auszulösen.

Dabei ist es vorteilhaft, dass die Umformung in mehreren Schritten erfolgt.

Erfindungsgemäß kann der metallische Werkstoff zur Herstellung von medizinischen Produkten, insbesondere zur Herstellung von Implantaten verwendet werden. Der Werkstoff kann ebenso zur Herstellung von Halbzeugen, insbesondere von Drähten, Rohren oder Bändern verwendet werden, die wiederum für die Herstellung von orthopädischen Implantaten, Herzschrittmacherkomponenten, Stimulationselektroden, Führungsdrähten oder Stents verwendet werden.

Nachfolgend wird die Erfindung beispielhaft erläutert.

Niob oder NbZr1 oder Tantal mit interstitiell gelöstem Sauerstoff können sehr gut als Grundstoff für zu implantierende medizinische Produkte verwendet werden. Bei Niob oder NbZr1 werden im vollständig rekristallisierten Materialzustand Druckfestigkeiten zwischen 750 MPa und 1.200 MPa bei Dehnungen im Bereich von 20 bis 30 % erreicht, wenn sie einen Sauerstoffgehalt von mindestens 1.000 µg/g aufweisen. Im Vergleich hierzu sind mit nicht Sauerstoff beladenem Niob oder NbZr1 im rekristallisierten Zustand lediglich Festigkeiten zwischen 275 MPa und 350 MPa bei vergleichbaren Dehnungswerten erreicht. Werden diese Werkstoffe verformt, zeigen sie eine hohe Festigkeit bei nur geringen Dehnungswerten (beispielsweise etwa 700 MPa bei einer Dehnung < 1 %).

## Patentansprüche

1. Verwendung eines metallischen Werkstoffs aus Niob oder einer Legierung auf Basis von Niob, der einen Sauerstoffgehalt von 1.000 bis 30.000 µg/g aufweist, wobei der Sauerstoff interstitiell in der Gitterstruktur des Metalls eingelagert ist für Halbzeuge, nämlich Rohre oder Bänder, für die Herstellung von orthopädischen Implantaten, Herzschrittmacherkomponenten oder Stents.

2. Verwendung nach Anspruch 1, **dadurch gekennzeichnet, dass** der Sauerstoff homogen verteilt eingelagert ist

3. Verwendung nach Anspruch 1 oder 2, **dadurch gekennzeichnet, dass** der Werkstoff einen Sauerstoffgehalt von 1.000 bis 15.000, vorzugsweise bis 8.000 µg/g aufweist.

4. Verwendung nach einem der Ansprüche 1 bis 3, **dadurch gekennzeichnet, dass** der Werkstoff mit einem oder mehreren Elementen aus der Gruppe H, C, N, P, S, V, Y, La, Hf, Ce, Th dotiert ist.

## Claims

1. Use of a metallic material of niobium or a niobium-based alloy, which material has an oxygen content of 1,000 to 30,000 µg/g, the oxygen being incorporated interstitially into the lattice structure of the metal, for semifinished products, i.e. tubes or tapes, for the production of orthopaedic implants, cardiac pacemaker components or stents.

2. Use according to claim 1 **characterised in that** the oxygen is incorporated in a homogeneously distributed manner.

3. Use according to claim 1 or 2 **characterised in that** the material has an oxygen content of 1,000 to 15,000, preferably of up to 8,000 µg/g.

4. Use according to one of claims 1 to 3 **characterised in that** the material is doped with one or several elements from the group of H, C, N, P, S, V, Y, La, Hf, Ce, Th.

## Revendications

1. Utilisation d'un matériau métallique en niobium ou en un alliage à base de niobium qui présente une teneur en oxygène de 1 000 à 30 000 µg/g, où l'oxygène est inclus de manière interstitielle dans la structure réticulaire du métal, pour des semi-produits, à savoir des tubes ou des bandes, pour la production d'implants orthopédiques, de composants de stimulateurs cardiaques ou de stents.

2. Utilisation selon la revendication 1 **caractérisée en ce que** l'oxygène est inclus en étant réparti de manière homogène.

3. Utilisation selon la revendication 1 ou 2 **caractérisée en ce que** le matériau présente une teneur en oxygène de 1 000 à 15 000, de préférence de jusqu'à 8 000 µg/g.

4. Utilisation selon l'une des revendications 1 à 3 **caractérisée en ce que** le matériau est dopé avec un ou plusieurs éléments du groupe H, C, N, P, S, V, Y, La, Hf, Ce, Th.
